# EUROPEAN PATENT APPLICATION

(11) **EP 4 407 033 A1**
(43) Date of publication of application: **31.07.2024**
(21) Application number: 22886021.9
(22) Date of filing: 26.10.2022
(51) Int. Cl.: C12N 15/63, C12N 15/62, C07K 1/22, C07K 19/00

(54) **METHOD FOR PURIFYING SINGLE-STRANDED DNA**

(30) Priority: 26.10.2021 CN 202111247093
(71) Applicant: Nanjing GenScript Biotech Co., Ltd., Nanjing, Jiangsu 211100 (CN)
(72) Inventor: CHEN, Juan, Nanjing, Jiangsu 211100 (CN); YE, Lumeng, Nanjing, Jiangsu 211100 (CN)
(74) Representative: Wang, Bo
(86) International application number: PCT/CN2022/127682
(87) International publication number: WO 2023/072145

(57) **Abstract**

A method for purifying single-stranded DNA is provided. The method can comprise: mixing a sample containing single-stranded DNA with a single-stranded DNA binding protein, and incubating for a first time period to obtain a first product, the first product containing a conjugate of the single-stranded DNA and the single-stranded DNA binding protein; purifying the first product by using affinity chromatography to obtain a second product; and purifying the second product to at least remove the single-stranded DNA binding protein to obtain a target product containing a purified single-stranded DNA. Also provided are a kit for purifying single-stranded DNA, said kit comprising a single-stranded DNA binding protein and a purification reagent; and a use of a single-stranded DNA binding protein in purifying single-stranded DNA.

## Description

### Cross Reference to Related Application

The present application claims priority to Chinese patent application No. 202111247093.4, filed on October 26, 2021, which is incorporated herein by reference in its entirety.

### Technical Field

The present application relates to the field of biotechnology, and in particular, to a method for purifying single-stranded DNA.

### Background Art

CRISPR/Cas9 is a technology for directionally engineering a DNA sequence of a target gene by a single-stranded gRNA (Guide RNA)-mediated Cas9 nuclease, and can efficiently achieve functions of specific gene knockout, gene knock-in, site-specific modification and the like. Single-stranded DNA serving as an HDR (Homology directed repair) template has a plurality of advantages in CRISPR/Cas9 gene knock-in experiments, such as improvement of editing efficiency and editing accuracy, and reduction of off-target effect and cytotoxicity. Therefore, the single-stranded DNA template is very suitable for being applied to insertion, replacement and correction of genes based on CRISPR technology, and is an ideal technical solution for gene editing of primary cells and stem cells and establishment of a gene engineering animal model.

At present, there are some methods capable of preparing a single-stranded DNA sample, for example, Patent WO 2020135651 discloses preparing a single-stranded DNA sample by skillfully combining uracil specific excision reagent (USER)-mediated self-looping of double-stranded DNA with accurate cleaving of a type II restriction endonuclease and rolling circle replication (RCA) of thermostatically amplified single-stranded DNA, which also increases the yield of a single-stranded DNA sample from conventional several ug to tens of ug. For another example, Veneziano R *et al.* disclose a method of preparing a single-stranded DNA template sample by asymmetric PCR (see Veneziano R, Shepherd T R, Ratanalert S, et al. In vitro synthesis of gene-length single-stranded DNA[J]. Scientific reports, 2018, 8(1): 1-7.). However, with the increasing demand, these technologies still have obvious drawbacks, such as the occurrence of non-specific amplification products in the reaction process, which often requires gel recovery for purification. This greatly increases the time and labor cost of the downstream purification process, hindering the large-scale preparation process. Therefore, there is a need for a method that can efficiently purify single-stranded DNA on a large scale.

### Summary

One of embodiments according to the present disclosure provides a method for purifying single-stranded DNA. The method comprises: mixing a sample containing the single-stranded DNA with a single-stranded DNA binding protein, and incubating for a first time period to obtain a first product, wherein the first product contains a conjugate of the single-stranded DNA and the single-stranded DNA binding protein; and purifying the first product to obtain a target product containing purified single-stranded DNA.

In some embodiments, when the sample is mixed with the single-stranded DNA binding protein, the single-stranded DNA binding protein has a final concentration of 0.5 ng/µL to 500 ng/µL.

In some embodiments, when the sample is mixed with the single-stranded DNA binding protein, the single-stranded DNA binding protein has a final concentration of 1 ng/µL to 200 ng/µL.

In some embodiments, the first time period is 30 minutes to 12 hours.

In some embodiments, the first time period is 2-4 hours.

In some embodiments, the sample is mixed with the single-stranded DNA binding protein and then incubated at 30-37°C.

In some embodiments, the step of purifying the first product comprises: separating and purifying the first product to obtain a second product; and purifying the second product to at least remove the single-stranded DNA binding protein, so as to obtain the target product containing purified single-stranded DNA.

In some embodiments, the first product is separated and purified by affinity chromatography, ion exchange chromatography or gel filtration chromatography.

In some embodiments, the first product is separated and purified by the affinity chromatography.

In some embodiments, the single-stranded DNA binding protein carries a purification tag.

In some embodiments, the purification tag is a polyhistidine tag, a glutathione-S-transferase tag, a FLAG tag or a Strep-tagII tag.

In some embodiments, the second product is purified by a process selected from at least one of the following: phenol chloroform extraction, DNA adsorption spin column recovery, plasmid extraction ion exchange column recovery, isopropanol precipitation, ethanol precipitation and molecular sieve chromatography.

In some embodiments, the method further comprises performing PCR amplification and DNA sequencing on the target product to verify whether the target product contains a complete sequence of the single-stranded DNA.

In some embodiments, the single-stranded DNA binding protein comprises a single-stranded DNA binding protein derived from *Escherichia coli,* phage phi29, *Salmonella* phage Vi II-E1, *Thermus thermophilus* HB8, *Klebsiella* phage Phi K02, *Yersinia* phage Py54 or *Synechococcus* phage S-CBS2, a core functional fragment thereof, a homolog sequence thereof, or a mutant sequence thereof.

In some embodiments, the single-stranded DNA binding protein comprises a sequence that has at least 80% sequence identity with an amino acid sequence set forth in SEQ ID NO: 2, 9, 10 or 11 and has a function of binding to single-stranded DNA.

One of embodiments according to this specification provides a kit for purifying single-stranded DNA, the kit comprises a single-stranded DNA binding protein and a purification reagent.

In some embodiments, the purification reagent is an affinity chromatography purification reagent.

One of embodiments according to the present disclosure provides a use of a single-stranded DNA binding protein in purifying single-stranded DNA.

### Brief Description of the Drawings

The present disclosure will be further illustrated by way of exemplary embodiments, and these exemplary embodiments will be described in detail with reference to the accompanying drawings. These embodiments are not limiting, wherein:
FIG. 1 is a schematic structural diagram of a recombinant expression vector according to some embodiments of the present disclosure;
FIG. 2 is an SDS-PAGE and Western Blot analysis pattern of a single-stranded DNA binding protein under a reducing condition according to some embodiments of the present disclosure;
FIG. 3 is a comparative agarose gel electrophoresis diagram before and after purification of single-stranded DNA of a target sequence 1 according to some embodiments of the present disclosure;
FIG. 4 is a schematic diagram of Sanger sequencing verification results of a target sequence 1 according to some embodiments of the present disclosure;
FIG. 5 is a comparative agarose gel electrophoresis diagram before and after purification of single-stranded DNA of a target sequence 2 according to some embodiments of the present disclosure; and
FIG. 6 is a schematic diagram of Sanger sequencing verification results of a target sequence 2 according to some embodiments of the present disclosure.

### Detailed Description

For a clearer description of the technical solutions in the embodiments of the present disclosure, the accompanying drawings required for describing the embodiments will be briefly described below. Apparently, the accompanying drawings in the following description show merely some examples or embodiments of the present disclosure, and those of ordinary skill in the art may still apply the present disclosure to other similar scenarios according to these accompanying drawings without any creative effort. Unless obvious from the linguistic context or otherwise stated, the same reference signs in the drawings represent the same structure or operation.

It should be understood that "device" or "unit" as used herein is a method for distinguishing different components, elements or parts of different levels. However, these words may be substituted by other expressions if these expressions accomplish the same purpose.

As shown in this specification and the claims, the words "one", "a", "an" and/or "the" do not specifically refer to the singular, but may also include the plural, unless the context clearly indicates otherwise. Generally, the terms "including" and "comprising" only imply the inclusion of explicitly identified steps and elements, and these steps or elements do not constitute an exclusive list. A method may further comprise other steps or elements.

A single-stranded DNA binding proteins (SSB) is a protein that can bind to single-stranded DNA, and mainly functions as stabilizing the unwound single strand of DNA during DNA replication, preventing renaturation and protecting the single-stranded part from being degraded by nucleases. This specification provides that the binding ability of the single-stranded DNA binding protein to single-stranded DNA can be used to purify single-stranded DNA in a large scale and efficiently to achieve higher yields.

According to one aspect of the present disclosure, a method for purifying single-stranded DNA is provided.

In some embodiments, the method comprises: mixing a sample containing single-stranded DNA with a single-stranded DNA binding protein, and incubating for a first time period to obtain a first product, wherein the first product contains a conjugate of the single-stranded DNA and the single-stranded DNA binding protein; purifying the first product to obtain a target product containing the purified single-stranded DNA.

In some embodiments, the sample may be a product of a process for large-scale production of single-stranded DNA, and thus the sample may contain non-specific amplification products, such as double-stranded DNA. In order to remove double-stranded DNA impurities, the conventional method is to use gel electrophoresis to recover single-stranded DNA in the sample. However, this method has a low yield, greatly increases the time and labor cost of a downstream purification process, and impedes the large-scale preparation. The purification method based on the single-stranded DNA binding protein provided by the present disclosure can be used to purify ] the single-stranded DNA in a large scale and efficiently by using the binding ability of the single-stranded DNA binding protein to the single-stranded DNA.

In some embodiments, the sample refers to any sample containing single-stranded DNA of a certain purity. A proportion of the single-stranded DNA in the sample may be about 10%, 20%, 30%, 40%, 50%, 60%, 70%, 80%, 90%, and the like, which is not limited by the present disclosure. The single-stranded DNA is also referred to as a target sequence.

In some embodiments, when the sample is mixed with the single-stranded DNA binding protein, the single-stranded DNA binding protein may have a final concentration of 0.1 ng/µL to 1000 ng/µL. In some embodiments, when the sample is mixed with the single-stranded DNA binding protein, the single-stranded DNA binding protein may have a final concentration of 0.5 ng/µL to 500 ng/µL. In some embodiments, when the sample is mixed with the single-stranded DNA binding protein, the single-stranded DNA binding protein may have a final concentration of 1 ng/µL to 200 ng/µL.

In some embodiments, the first time period of incubation after the sample is mixed with the single-stranded DNA binding protein may be 20 minutes to 24 hours. In some embodiments, the first time period of incubation after the sample is mixed with the single-stranded DNA binding protein may be 30 minutes to 15 hours. In some embodiments, the first time period of incubation after the sample is mixed with the single-stranded DNA binding protein may be 30 minutes to 12 hours, for example, 2 hours to 4 hours. In some embodiments, the first time period of incubation after the sample is mixed with the single-stranded DNA binding protein may be 30 minutes to 6 hours. In some embodiments, the first time period of incubation after the sample is mixed with the single-stranded DNA binding protein may be 2-4 hours.

In some embodiments, the sample is mixed with the single-stranded DNA binding protein and then incubated at 30-37°C. For illustration only, the incubation temperature may be 30°C, 32°C, 35°C, 37°C, and the like.

In some embodiments, the step of purifying the first product may comprise: separating and purifying the first product to obtain a second product; and purifying the second product to at least remove the single-stranded DNA binding protein to obtain the target product containing purified single-stranded DNA.

In some embodiments, the first product is separated and purified to remove impurities in the first product that do not bind to the single-stranded DNA binding protein, and thus a second product is obtained. This step can be implemented by various methods for separating and purifying proteins. For example, the purification method may include affinity chromatography, ion exchange chromatography, gel filtration chromatography (molecular sieve) or the like, which is not limited by the present disclosure. In some embodiments, the method for separating and purifying the first product is affinity chromatography. In some embodiments, the affinity chromatography may include various affinity chromatography methods for separating and purifying proteins. This is not limited by the present disclosure.

In some embodiments, the single-stranded DNA binding protein may further comprise a purification tag for affinity chromatography purification, such as a polyhistidine tag, a glutathione-S-transferase tag, a FLAG tag or a Strep-tagII tag.

For example only, the single-stranded DNA binding protein may carry a purification tag, and magnetic beads corresponding to the purification tag may be used for purification. For example, a single-stranded DNA binding protein may comprise a polyhistidine tag, and the corresponding magnetic beads may carry nickel; the single-stranded DNA binding protein may comprise a glutathione-S-transferase (GST) tag, and the corresponding magnetic beads may be GST fusion protein purification magnetic beads; or the single-stranded DNA binding protein may comprise a Strep-tagII tag, and the corresponding magnetic beads may be Strep-tagII protein purification magnetic beads. For another example, the single-stranded DNA binding protein may use a FLAG tag, and a FLAG-tag fusion protein purification kit may be used for purification.

In some embodiments, the first product and an affinity chromatography purification medium may be incubated at room temperature for 30 minutes to 1 hour, and components that do not bind to the single-stranded DNA binding protein are eluted by a buffer with different concentration gradients (e.g., a buffer containing imidazole with different concentrations) or a buffer with different pH gradients to obtain a second product containing a conjugate of single-stranded DNA and the single-stranded DNA binding protein. This method is suitable for large-scale purification of single-stranded DNA, and has the advantages of a high yield, high speed and low cost (see Example 4). The whole purification process does not involve a DNA dye and a carcinogenic agent, and the product has good biological safety, which provides great convenience for clinical trials and other applications. In addition, the purification process of the final product does not involve amplification or denaturation, and nucleotide errors, losses or mutation caused by high-difficulty sequences in the target sequence can be avoided, so that the method for purifying the single-stranded DNA provided by the present disclosure can be suitable for purifying single-stranded DNA with various lengths and structures, and has a wide application range. The reagent materials required in the whole process are conventional protein purification reagents and tools, the system is convenient to amplify, and the cost is low. The term "high-difficulty sequence" refers to a sequence that contains, but is not limited to, hairpin structure which is caused by inverted repeats, high-low GC sequence or poly structure, which are difficult to amplify by PCR, resulting in inefficient synthesis.

In some embodiments, in the process of affinity chromatographypurification, after the affinity adsorption step is completed, the remaining liquid may be optionally collected and analyzed to determine whether the single-stranded DNA is well adsorbed to the affinity adsorption medium. For example, if affinity chromatography is performed using a magnetic bead method, the magnetic beads can be collected using a magnetic separation rack after incubating the first product with the magnetic beads for 30-60 minutes, and the supernatant is obtained for analysis. For example only, a certain amount of supernatant may be taken and analyzed by gel electrophoresis. If the result shows that no to-be-purified single-stranded DNA exists in the supernatant, it indicates that the DNA in the sample is substantially completely adsorbed on the affinity adsorption medium. If the result shows that a large amount of single-stranded DNA still exists in the supernatant, it indicates that the single-stranded DNA binding protein does not completely bind to the single-stranded DNA in the sample, or the selected affinity chromatography medium has a poor effect. In this case, the purification yield can be improved by examining whether the used single-stranded DNA binding protein can normally bind to single-stranded DNA, increasing the concentration of the used single-stranded DNA binding protein, increasing the incubation time period after the sample is mixed with the single-stranded DNA binding protein, or replacing the affinity chromatography medium.

In some embodiments, after the affinity chromatography purification step is completed, the second product can optionally be sampled for analysis to determine the effect of the affinity chromatography purification. For example, a certain amount of the second product may be taken and analyzed by gel electrophoresis. If the result shows that only single-stranded DNA exists in the second product, it indicates that the double-stranded DNA impurities are removed; if the result shows that both single-stranded DNA and double-stranded DNA exist in the second product, it indicates that the double-stranded DNA impurities are not completely removed, and the selected affinity chromatography process is not suitable. For example, the non-specific adsorption of the double-stranded DNA may be caused by the excessive amount of the single-stranded DNA binding protein, which can be improved by reducing the concentration of the used single-stranded DNA binding protein, reducing the incubation time period after the sample is mixed with the single-stranded DNA binding protein, or performing secondary incubation and purification on the sample and a small amount of the single-stranded DNA binding protein, so as to avoid the situation that the double-stranded DNA impurities are not completely removed.

In some embodiments, the second product is purified to remove single-stranded DNA binding protein, imidazole, salts and other undesirable impurities from the second product. This purification step may be selected from any one or more of the following methods: phenol chloroform extraction, DNA adsorption spin column recovery, plasmid extraction ion exchange column recovery, isopropanol precipitation, ethanol precipitation and molecular sieve chromatography supported by high performance liquid chromatography. This is not limited by the present disclosure.

In some embodiments the single-stranded DNA binding protein comprises a single-stranded DNA binding protein derived from *Escherichia coli,* phage phi29 (*Bacillus* phage *Nf*), *Salmonella* phage Vi II-E1, *Thermus thermophilus* HB8, *Klebsiella* phage Phi K02, *Yersinia* phage Py54 or *Synechococcus* phage S-CBS2, a core functional fragment thereof, a homolog sequence thereof, or a mutant sequence thereof. The term "core functional fragment" as used herein refers to a fragment in the protein structure that enables the protein to normally complete its specific function. The term "homolog sequence" refers to a sequence that derives from the same ancestor but that gradually develops partial differences during evolution. The "mutant sequence" refers to a sequence after natural mutation or artificially induced mutation of a wild-type sequence. The core functional fragment, the homolog sequence, or the mutant sequence mentioned here derived from of the single-stranded DNA binding proteins of the above-mentioned microorganisms still have the ability to bind to single-stranded DNA. It should be noted that the single-stranded DNA binding protein used in the present disclosure may be a protein from various sources and capable of binding to single-stranded DNA, such as derived from other microorganisms not mentioned above or derived from artificial synthesis. This is not limited by the present disclosure.

In some embodiments, the single-stranded DNA binding protein is a mutant of the single-stranded DNA binding protein from the above-described microorganism; and the mutant of the single-stranded DNA binding protein comprises a sequence that has at least 70%, 75%, 80%, 85%, 90%, 91%, 92%, 93%, 94%, 95%, 96%, 97%, 98% or 99% of sequence identity to an amino acid sequence of the single-stranded DNA binding protein from the above-mentioned microorganism and has a function of binding to single-stranded DNA.

For example only, the single-stranded DNA binding protein derived from *Thermus thermophilus* HB8 has accession No. WP_011227803.1 at NCBI (SEQ ID NO: 9); the mutant derived from the single-stranded DNA binding protein from *Thermus thermophilus* HB8 has an amino acid sequence of SEQ ID NO: 2 and a nucleotide sequence of SEQ ID NO: 1. the single-stranded DNA binding protein derived from *Escherichia coli* K-12 has accession No. AKK14256.2 (SEQ ID NO: 10) at NCBI; and the single-stranded DNA binding protein derived from phage *phi29* has accession No. YP_009910721.1 at NCBI (SEQ ID NO: 11). In some embodiments, the single-stranded DNA binding protein comprises a sequence that has at least 85%, 90%, 91%, 92%, 93%, 94%, 95%, 96%, 97%, 98% or 99% of sequence identity to an amino acid sequence set forth in SEQ ID NO: 2, 9, 10 or 11 and has a function of binding to single-stranded DNA. In some embodiments, the single-stranded DNA binding protein comprises an amino acid sequence set forth in SEQ ID NO: 2, 9, 10 or 11. In one specific embodiment, the amino acid sequence of the single-stranded DNA binding protein is set forth in SEQ ID NO: 2, 9, 10 or 11.

In some embodiments, the identity of the single-stranded DNA binding protein to the amino acid sequence set forth in SEQ ID NO: 2 is greater than or equal to 80%. In some embodiments, the identity of the single-stranded DNA binding protein to the amino acid sequence set forth in SEQ ID NO: 2 is greater than or equal to 85%. In some embodiments, the identity of the single-stranded DNA binding protein to the amino acid sequence set forth in SEQ ID NO: 2 is greater than or equal to 90%. In some embodiments, the identity of the single-stranded DNA binding protein to the amino acid sequence set forth in SEQ ID NO: 2 is greater than or equal to 92%. In some embodiments, the identity of the single-stranded DNA binding protein to the amino acid sequence set forth in SEQ ID NO: 2 is greater than or equal to 95%. In some embodiments, the identity of the single-stranded DNA binding protein to the amino acid sequence set forth in SEQ ID NO: 2 is greater than or equal to 97%. In some embodiments, the identity of the single-stranded DNA binding protein to the amino acid sequence set forth in SEQ ID NO: 2 is greater than or equal to 99%. In one embodiment, the amino acid sequence of the single-stranded DNA binding protein is set forth in SEQ ID NO. 2.

As used herein, sequence identity (also referred to as "Sequence Identity") refers to the amount of identity, generally expressed as a percentage, between two nucleotide/amino acid sequences (e.g., a query sequence and a reference sequence). Typically, sequence alignment is performed and a gap (if any) is introduced before calculating the percentage identity between two nucleotide/amino acid sequences. If bases or amino acids in the two sequences are the same at a certain alignment position, the two sequences are considered to be identical or matched at that position. If the bases/amino acids in the two sequences are different, the two sequences are considered to be non-identical or mismatched at that position. In some algorithms, the number of matched positions is divided by the total number of positions in the alignment window to obtain sequence identity. In other algorithms, the number of gaps and/or the gap length are also taken into account. For the purpose of the present disclosure, the publicly available alignment software BLAST (available at ncbi.nlm.nih.gov) can be employed to obtain the optimal sequence alignment and calculate the sequence identity between two nucleotide/amino acid sequences by using default settings.

In some embodiments, after obtaining the target product, optionally, the target product may be subj ected to PCR amplification and DNA sequencing to verify whether the target product contains a complete sequence of the single-stranded DNA. If the sequencing result is consistent with the sequence of the single-stranded DNA, it indicates that the single-stranded DNA in the target product is complete and can be used in the next step, including but not limited to CRISPR/Cas9 technology, to achieve functions of specific gene knockout, gene knock-in, site-specific modification, and the like. If the sequencing result is inconsistent with the sequence of the single-stranded DNA, it indicates that the sample may contain various single-stranded DNA or other impurities may be mixed in the purification process, and further purification is required.

According to another aspect of the present disclosure, a kit for purifying single-stranded DNA is provided. In some embodiments, the kit may comprise the aforementioned single-stranded DNA binding protein and protein purification reagent. In some embodiments, the protein purification reagent is an affinity chromatography purification reagent. For example, the affinity chromatography purification reagent may comprise a magnetic bead, an elution buffer, a wash buffer and a binding buffer. For example only, the binding buffer may contain 50 mM NaH₂PO₄ and 300 mM NaCl, and has a pH of 8.0. For another example, the wash buffer may contain 50 mM NaH₂PO₄, 300 mM NaCl and 10 mM imidazole, and has a pH of 8.0. For still another example, the elution buffer may contain 50 mM NaH₂PO₄, 300 mM NaCl and 250 mM imidazole, and has a pH of 8.0.

In some embodiments, the kit may further comprise one or more nucleic acid primers and one or more nucleic acid probes for PCR amplification and sequencing to verify whether the target product contains a complete sequence of the single-stranded DNA.

According to still another aspect of the present disclosure, a use of a single-stranded DNA binding protein in purifying single-stranded DNA is provided. For example, in some embodiments, the use comprises: mixing a sample containing the single-stranded DNA with a single-stranded DNA binding protein, and incubating for a first time period to obtain a first product, the first product contains a conjugate of the single-stranded DNA and the single-stranded DNA binding protein; purifying the first product by using affinity chromatography to obtain a second product; and purifying the second product to at least remove the single-stranded DNA binding protein to obtain a target product containing purified single-stranded DNA.

The method for purifying single-stranded DNA provided by the present disclosure may bring about beneficial effects including but not limited to: (1) the method is suitable for large-scale purification of single-stranded DNA, and has a high yield; (2) compared with a conventional method (such as gel purification), the method for purifying single-stranded DNA provided by the present disclosure uses an affinity chromatography process, and has high speed and high efficiency; (3) the whole purification process does not involve a DNA dye and a carcinogenic agent, and the product has good biological safety, which provides great convenience for clinical trials and other applications; (4) the purification process of the final product does not involve amplification or denaturation, and nucleotide errors, losses or mutation caused by high-difficulty sequences in the target sequence can be avoided, so that the method for purifying the single-stranded DNA provided by the present disclosure can be suitable for purifying single-stranded DNA with various lengths and structures, and has a wide application range; and (5) the reagent materials required in the whole process are conventional protein purification reagents and tools, the system is convenient to amplify, and the cost is low.

Unless otherwise stated, the experimental methods used in the following examples are conventional methods. The test materials used in the following examples were purchased from conventional biochemical reagent companies unless otherwise specified. Unless otherwise specified, the quantitative tests in the following examples were performed in triplicate, and the results were averaged.

### Example 1. Preparation of single-stranded DNA binding proteins

### 1. Gene synthesis and recombinant vector construction:

A mutant sequence (SEQ ID NO: 1) of an SSB gene derived from *Thermus thermophilus* HB8 selected in the present disclosure was synthesized by Nanjing GenScript Biotech Co., Ltd.

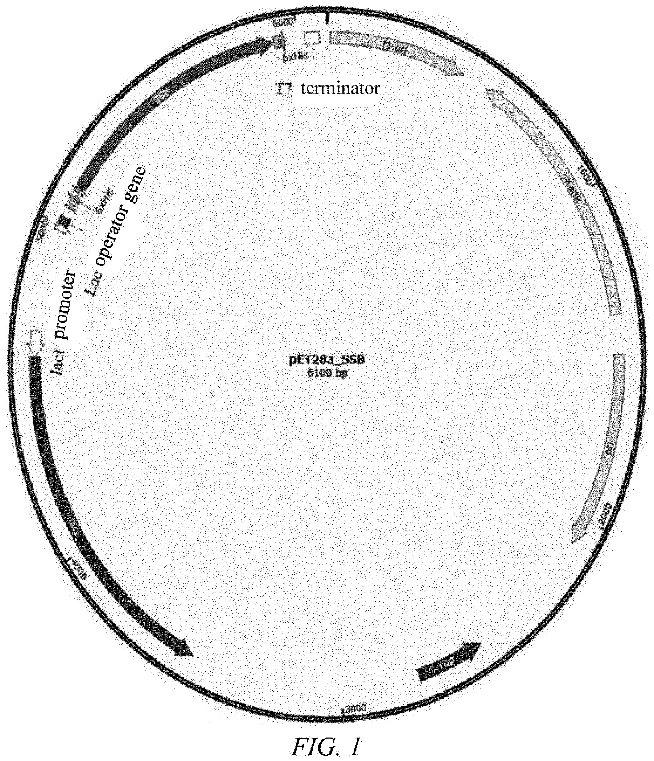

A pET28a kanamycin resistance vector was selected as an expression vector, an SSB gene (SEQ ID NO: 1) was inserted between restriction endonuclease cutting sites NdeI and HindIII, i.e., between base 5130 and base 5923 to obtain a protein expression vector having a polyhistidine tag at the N-terminus, and a recombinant plasmid was obtained by a homologous recombination method, as shown in FIG. 1. An amino acid sequence of the SSB protein obtained and encoded by the SSB gene mutant sequence is set forth in SEQ ID NO: 2.

### 2. Protein expression:

*Escherichia coli* BL21 (DE3) strain was transformed with the recombinant plasmid. A single colony was inoculated into a TB large-scale medium containing kanamycin resistance and cultured at 37°C. When OD₆₀₀ reached about 1.2, the cultured cells were induced to express target proteins with 0.5 mM IPTG at 15°C/16 h. The cells were harvested by centrifugation.

### 3. Purification and analysis:

The cell precipitate was resuspended in lysis buffer (lysis buffer: 50 mM Tris, 150 mM NaCl, 5% glycerol) and then sonicated, and the supernatant was collected after centrifugation. The supernatant was further purified by using a nickel-containing affinity chromatography purification medium column to obtain the target protein. The target protein was filtered and sterilized using a 0.22 µm filter membrane, and then stored in aliquots.

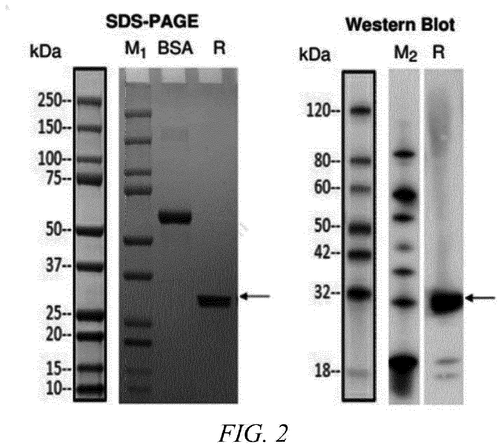

The protein concentration was determined by Bradford protein assay using BSA as a standard, and the purified single-stranded DNA binding protein had a concentration of 2.62 mg/ml. Protein purity and molecular weight were determined by reducing SDS-PAGE and Western blot (the main antibody involved was murine anti-His antibody, purchased from Nanjing GenScript Biotech Co., Ltd., Cat. No. A00186) results, as shown in FIG. 2, where lane M1 is protein Marker (Bio-rad, Cat. No.1610374S), lane M2 is protein Marker (Genscript, Cat. No.M00521), lane BSA is standard; and lane R is the purified target single-stranded DNA binding protein, and the band of the purified single-stranded DNA binding protein is consistent with the theoretical molecular weight (32.05 KDa) and the purity is 90%.

The gene synthesis and the recombinant vector construction, protein expression and purification analysis related to the above-mentioned methods are all completed by the gene synthesis service and the protein purification service of Nanjing GenScript Biotech Co., Ltd. (https://www.genscript.com.cn/guaranteed-bacterial-protein-expression-services.html?src=pullmenu).

### Example 2. Purification of target sequence 1

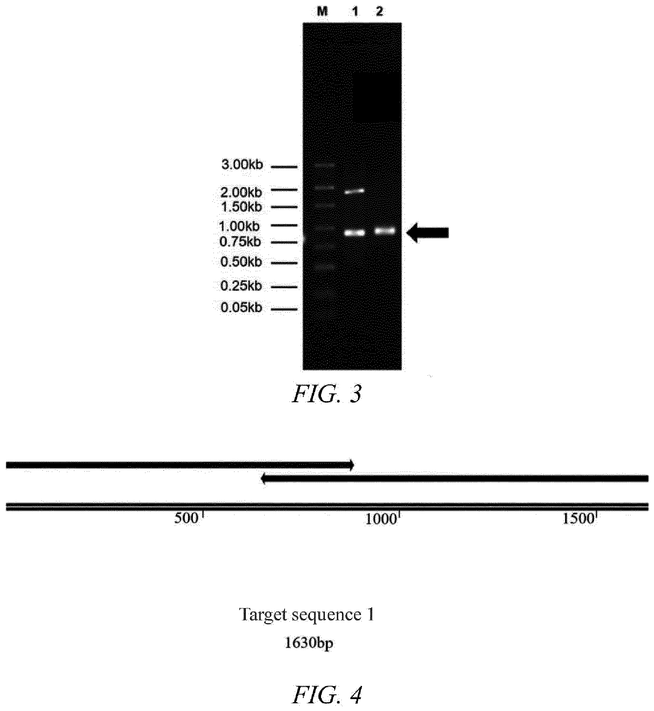

This example is intended for downstream purification of a crude single-stranded DNA product prepared by various methods, and the method for preparing single-stranded DNA is not described herein. According to the method disclosed in Patent WO 2020135651, single-stranded DNA was prepared by using rolling circle replication (RCA) of thermostatically amplified single-stranded DNA to obtain the target sequence 1: the crude single-stranded DNA product with a length of 1630 nt (SEQ ID NO: 3), containing many non-specific amplification products. The crude single-stranded DNA product was analyzed by agarose gel electrophoresis, as shown in FIG. 3, where lane 1 is the crude single-stranded DNA product, and the proportion of single-stranded DNA product in grayscale analysis is 60.25%.

The purification process of this example is as follows:
Step I: Binding of single-stranded DNA binding protein to crude single-stranded DNA product.

The purified single-stranded DNA binding protein obtained in Example 1 was diluted 5-fold, the diluted single-stranded DNA binding protein was added to 10 mL of crude single-stranded DNA product and mixed evenly, and the volume ratio of the mixture was shown in Table 1. The mixture was incubated for 2 hours at 37°C to ensure that the single-stranded DNA binding protein and the target single-stranded DNA in the crude product can be fully binded to obtain an incubation product.

**Table 1**

| Reagent | Unit (mL) |
|---|---|
| Crude single-stranded DNA product | 10 |
| Single-stranded DNA binding protein | 1 |

### Step II: Purification of the target sequence by affinity chromatography.

The Ni-Charged MagBeads magnetic bead (Ni filled magnetic beads, Nanjing GenScript Biotech Co., Ltd., Cat. No. L00295) purification method was used to purify the target sequence 1 that binds to the single-stranded DNA binding protein in step I to obtain an affinity chromatography purified product. The specific steps are as follows:
1) The Ni-Charged MagBead was resuspended completely by shaking or vortexing the vial.
2) 4 mL of magnetic beads were transferred to a clean tube.
3) The tube was placed on a magnetic separation rack to collect the magnetic beads, and the supernatant was decanted and discarded.
4) 10 mL of LE Buffer (50 mM NaH₂PO₄, 300 mM NaCl, pH 8.0) was added to the tube, and the tube was inverted several times for mixing. The beads were collected using a magnetic separation rack, and the supernatant was discarded. This step was repeated twice.
5) An equal volume of the incubation product from step I was added to the tube, and the tube was inverted gently for mixing.
6) Incubation was performed at 20°C for 30-60 minutes with shaking.
7) The magnetic beads were collected using a magnetic separation rack and the supernatant was discarded. If necessary, the supernatant was retained for analysis.
8) 10 ml of Wash Buffer (50 mM NaH₂PO₄, 300 mM NaCl, 10 mM imidazole, pH 8.0) was added to the tube, and the tube was inverted gently for mixing. The tube was placed on a magnetic separation rack to collect the magnetic beads, and the supernatant was decanted and discarded. The washing step was repeated twice.
9) 1 mL of Elution Buffer (50 mM NaH₂PO₄, 300 mM NaCl, 250 mM imidazole, pH 8.0) was added to the tube, mixed well, and incubated at 20°C for 5 minutes with shaking.
10) The tube was placed on a magnetic separation rack to collect the magnetic beads, and the supernatant containing the eluted protein was transferred to a clean tube.
11) Steps 9) and 10) were repeated twice if necessary. The supernatant was used as a sample for subsequent purification.
12) Agarose gel electrophoresis was performed on the supernatant (purified product) collected in the step 11), and whether the purification was complete was preliminarily determined through comparison and analysis of bands before and after the purification.

### Step III: Removal and purification of single-stranded DNA binding protein with polyhistidine tag.

The affinity chromatography purified product obtained in step II contained byproducts such as single-stranded DNA binding protein, imidazole and salt, and in this example, the byproducts such as protein, imidazole and salt in the reaction system were removed by using Qiagen-DNA plasmid extraction kit (QIAGEN, Cat. No./ID: 12143), and finally the purified product was obtained, as shown in FIG. 3, where lane 1 is a crude single-stranded DNA product, the proportion of single-stranded DNA in grayscale analysis is 60.25%, lane 2 is a purified single-stranded DNA product, and the proportion of single-stranded DNA in grayscale analysis is 100%, which indicates that the purified single-stranded DNA product of target sequence 1 with 100% purity was obtained through step III.

This step can also remove protease and salt in the reaction system by phenol-chloroform extraction, DNA adsorption spin column recovery, isopropanol/ethanol precipitation, or molecular sieve chromatography supported by high performance liquid chromatography.

### Step IV: Sanger sequencing verification.

The purified single-stranded DNA product obtained in step III was diluted to 50 ng/µL, and the purified single-stranded DNA product was used as a template. Primer 1630 nt-F and primer 1630 nt-R were used to amplify the full length sequence which was ligated into the universal vector pUC57 by means of blunt end manner.

The names and sequences of the amplification primers are as follows:
1630 nt-F: GGTTTCCTTGAGTGGCAGGC (SEQ ID NO: 4)
1630 nt-R: TGGCCATTCCTGAAGCAAGG (SEQ ID NO: 5)

The names and sequences of the sequencing primers are as follows:
M13F (47): CGCCAGGGTTTTCCCAGTCACGAC (SEQ ID NO: 6)
M13R (48): AGCGGATAACAATTTCACACAGGA (SEQ ID NO: 7)

Sanger sequencing was performed and the verification results are shown in FIG. 4. The verification result shows that the target single-stranded DNA sequence conforms to a theoretical sequence and no error site occurs, which indicates that this method is a practical, efficient and convenient method for purifying the target single-stranded DNA in a large scale.

### Example 3. Purification of target sequence 2

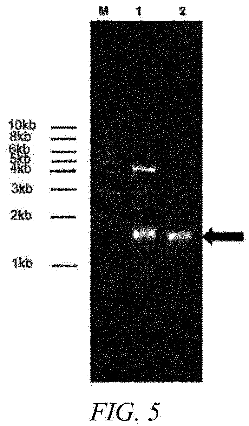

According to the method disclosed in Patent WO 2020135651 single-stranded DNA was prepared by using rolling circle replication (RCA) of thermostatically amplified single-stranded DNA to obtain the target sequence 2: A crude single-stranded DNA product (SEQ ID NO: 8) with a length of 4000 nt, containing many non-specific amplification products. The crude single-stranded DNA product was analyzed by agarose gel electrophoresis, as shown in FIG. 5. Lane 1 is a crude single-stranded DNA product, and the proportion of single-stranded DNA in grayscale analysis is 53.45%.

The purification process of this example is as follows:
Step I: Binding of single-stranded DNA binding protein to crude single-stranded DNA product

The purified single-stranded DNA binding protein obtained in Example 1 was diluted 5-fold, the diluted single-stranded DNA binding protein was added to 10 mL of crude single-stranded DNA product and mixed evenly, and the volume ratio of the mixture was shown in Table 1. The mixture was incubated for 2 hours at 37°C to ensure that the single-stranded DNA binding protein and the target single-stranded DNA in the crude product can be fully binded to obtain an incubation product.

### Step II: Purification of the target sequence by affinity chromatography.

The Ni-Charged MagBeads magnetic bead (Nanjing GenScript Biotech Co., Ltd., Cat. No. L00295) purification method was used to purify the target sequence 2 that binds to the single-stranded DNA binding protein in step I. The specific steps are as follows:
1) The Ni-Charged MagBead was resuspended completely by shaking or vortexing the vial.
2) 4 mL of magnetic beads were transferred to a clean tube.
3) The tube was placed on a magnetic separation rack to collect the magnetic beads, and the supernatant was decanted and discarded.
4) 10 mL of LE Buffer (50 mM NaH₂PO₄, 300 mM NaCl, pH 8.0) was added to the tube, and the tube was inverted several times for mixing. The beads were collected using a magnetic separation rack, and the supernatant was discarded. This step was repeated twice.
5) An equal volume of the incubation product from step I was added to the tube, and the tube was inverted gently for mixing.
6) Incubation was performed at 20°C for 30-60 minutes with shaking.
7) The magnetic beads were collected using a magnetic separation rack and the supernatant was discarded. If necessary, the supernatant was retained for analysis.
8) 10 ml of Wash Buffer (50 mM NaH₂PO₄, 300 mM NaCl, 10 mM imidazole, pH 8.0) was added to the tube, and the tube was inverted gently for mixing. The tube was placed on a magnetic separation rack for several minutes to collect the magnetic beads, and the supernatant was decanted and discarded. The washing step was repeated twice.
9) 1 mL of Elution Buffer (50 mM NaH₂PO₄, 300 mM NaCl, 250 mM imidazole, pH 8.0) was added to the tube, mixed well, and incubated at 20°C for 5 minutes with shaking.
10) The tube was placed on a magnetic separation rack for several minutes to collect the magnetic beads, and the supernatant containing the eluted protein was transferred to a clean tube.
11) Steps 9) and 10) were repeated twice if necessary, and the supernatant was used as a sample for subsequent purification.
12) Agarose gel electrophoresis was performed on the supernatant (purified product) collected in the step 11), and whether the purification was complete was preliminarily determined through comparison and analysis of bands before and after the purification.

### Step III: Removal and purification of single-stranded DNA binding protein with polyhistidine tag.

The affinity chromatography purified product contained the added single-stranded DNA binding protein, imidazole, salt and other products, and in this example, the removal of protease and salt in the reaction system was performed by a Qiagen-DNA plasmid extraction column. The purified single-stranded DNA product of the target sequence 2 was finally obtained, as shown in FIG. 5, where lane 1 is a crude single-stranded DNA product, the proportion of single-stranded DNA in grayscale analysis is 53.45%, lane 2 is a purified single-stranded DNA product, and the proportion of single-stranded DNA in grayscale analysis is 100%, which indicates that the purified single-stranded DNA product of target sequence 2 with 100% purity was obtained through step III.

This step can also remove protease and salt in the reaction system by phenol-chloroform extraction, DNA adsorption spin column recovery, isopropanol/ethanol precipitation, or molecular sieve chromatography supported by high performance liquid chromatography.

### Step IV: Sanger sequencing verification.

The purified single-stranded DNA product of target sequence 2 obtained in step III was diluted to 50 ng/µL. The purified single-stranded DNA product of target sequence 2 was used as a template, primer P-4000-F and primer P-4000-seq4 were used to amplify the 1-2000 bp region of the sequence, and primer P-4000-seq11 and primer P-4000-R were used to amplify the 1788-4000 bp region of the sequence, and the amplified sequences were ligated into the universal vector pUC57 by means of blunt end manner, respectively.

The names and sequences of the amplification primers are as follows:
p4000-F: GGTATCAGCTCACTCAAAGG (SEQ ID NO: 12)
p4000-R: CTGTCTCTTGATCAGATCCG (SEQ ID NO: 13)
p4000-seq4: CACTACTCAGCGACCTCCAA (SEQ ID NO: 14)
p4000-seq11: ATTGTCTCATGAGCGGATAC (SEQ ID NO: 15)

The names and sequences of the sequencing primers are as follows:
M13F (47): CGCCAGGGTTTTCCCAGTCACGAC (SEQ ID NO: 6)
M13R (48): AGCGGATAACAATTTCACACAGGA (SEQ ID NO: 7)
p4000-seq4: CACTACTCAGCGACCTCCAA (SEQ ID NO: 14)
p4000-seq5: GGGCAAGAGCAACTCGGTCG (SEQ ID NO: 16)
p4000-seq9: CCTAACTCCGCCCAGTTCCG (SEQ ID NO: 17)
p4000-seq10: ATAGGGAGACCCAAGCTGGC (SEQ ID NO: 18)

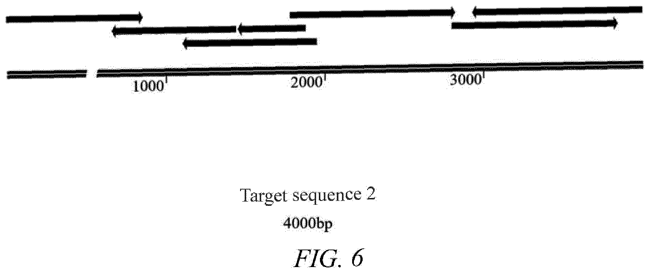

Sanger sequencing was performed and the verification results are shown in FIG. 6. The verification result shows that the target single-stranded DNA sequence conforms to a theoretical sequence and no error site occurs, which indicates that this method is a practical, efficient and convenient method for purifying the target sequence in a large scale.

### Example 4. Comparison of conventional gel electrophoresis and the purification method provided in this specification

Taking the purification of 50 mL of crude single-stranded DNA product of target sequence 1 as an example, the results of the purification method using conventional agarose gel electrophoresis recovery and the purification method using single-stranded DNA binding protein provided in the present disclosure were compared. The specific steps are as follows:

### 1. Conventional agarose gel electrophoresis

The conventional agarose gel electrophoresis recovery uses an AxyPrep DNA gel recovery kit (Axygen, Cat. No. AP-GX-250) for purification, and the specific steps are as follows:
Step I: Preparation of 2.5% agarose gel electrophoresis
1) A gel-making tool was washed with distilled water, a gel-making flat plate was prepared, the edge of a mold was sealed with agar, and a comb was erected.
2) A 2.5% agarose gel was prepared according to a size of and a volume after dilution of to-be-separated samples (1630 nt ssDNA and dsDNA). For example, 1 piece of agarose gel was prepared for 1.5 mL of sample, and 67-100 pieces of agarose gel were prepared according to a loading volume (about 100 mL to 150 mL after dilution).
3) The melted agar solution was shaken and mixed uniformly, poured into an electrophoresis tank, and solidified.
4) The agar solution was solidified for about 30-40 min at room temperature, the comb was carefully pulled out, the solidified gel was taken out and put into an electrophoresis tank and was ready for sample loading.

### Step II: Gel electrophoresis of crude single-stranded DNA product

1) An electrophoresis buffer (TAE) was poured into an electrophoresis tank, and submerged the gel surface by about 1 mm; and bubbles in the gel wells were removed.
2) Sample dilution: To better distinguish between ssDNA and dsDNA, 50 ml of the crude product was diluted 2-3 fold with water. The dye for gel recovery was added according to a proportion of adding 1 ul of the dye for gel recovery into 19 µL of the sample, uniformly mixed by using a pipette, and added into the gel wells.
3) After the loading was completed, a power supply was turned on, where the red represented positive electrode and the black represented negative electrode, the DNA sample moved from the negative electrode to the positive electrode, the side of the sample adding well was negative electrode, and this process was operated at 150 V-220 V for 30-40 min.
4) After the voltage was over, the power supply was turned off, and the gel imager was used to observe whether the ssDNA and dsDNA were completely separated.

### Step III: Gel purification

1) Gel cutting (cut by a clean blade without nucleic acid contamination): After electrophoresis, agarose gel corresponding to the size of the single-stranded DNA fragment was cut under an ultraviolet lamp, wherein every three pieces of agarose gel was placed in a 50 ml centrifuge tube.
2) About 7.5 ml of Buffer DE-A was added to each 50 ml centrifuge tube, mixed uniformly and heated at 75°C with intermittent mixing (every 2-3 min) until the gel mass was completely melted (about 30 min to 1 h for three gels).
3) 3.75 ml of Buffer DE-B was added and mixed uniformly.
4) 700 µL of the mixed solution in the previous step was pipetted and transferred to a DNA preparation tube (placed in a 2 ml centrifuge tube provided in the kit) for a total of about 1000 preparation tubes. Centrifugation was performed at 12,000×g for 1 min. The filtrate was transferred again to the original preparation tube and centrifuged at 12,000×g for 1 min.
5) The preparation tubes were placed back in 2 ml centrifuge tubes, 500 µl of Buffer W1 was added to each preparation tube and centrifugation was performed at 12,000×g for 30 s, and the filtrate was discarded.
6) The preparation tubes were placed back in 2 ml centrifuge tubes, 700 µl of Buffer W2 was added to each preparation tube and centrifugation was performed at 12,000×g for 30 s, and the filtrate was discarded. According to the same procedure, each preparation tube was washed once more with 700 µl of Buffer W2 and centrifugation was performed at 12,000×g for 1 min.
7) The preparation tubes were placed back in 2 ml centrifuge tubes, centrifuged at 12,000×g for 1 min, and left to stand for 30 min.
8) The preparation tubes were placed in clean 1.5 ml centrifuge tubes (provided in the kit), 25-30 µL deionized water was added to the center of the preparation membrane, and the membrane was allowed to stand at room temperature for 5 min. The target single-stranded DNA was eluted by centrifugation at 12,000×g for 1 min.

### 2. Purification method using single-stranded DNA binding protein

The procedure of Example 2 was followed, with the reagent addition scaled up in a ratio of 1 : 5, and the remaining parameters unchanged.

Taking the purification of 50 mL of crude single-stranded DNA product of target sequence 1 as an example, the differences in the time period, cost and yield of the target product between the purification method using conventional agarose gel electrophoresis recovery and the purification method using single-stranded DNA binding protein provided in the present disclosure were recorded, as shown in Table 2.

**Table 2**

| | Purification method using agarose gel electrophoresis | Purification method using single-stranded DNA binding protein |
|---|---|---|
| Time period (h) | 19 | 5 |
| Reagent cost ( ¥ ) | 2500 | 820.29 |
| Yield (µg) | 260 | 965 |

The result shows that, compared with the conventional purification method using agarose gel electrophoresis recovery, the purification method using single-stranded DNA binding protein shortens 73.7% of the time period; reduces by 67.2% of the cost of the reagents used, and increases by a factor of 2.7 of the yield of the desired product.

It should be understood by those skilled in the art that the above embodiments are only for illustrating the present disclosure, and do not limit the present disclosure. Any modifications, equivalent substitutions, changes, and the like made within the spirit and principle of the present disclosure should fall within the scope of protection of the present disclosure.

## Claims

1. A method for purifying single-stranded DNA, **characterized in that** the method comprises:
mixing a sample containing the single-stranded DNA with a single-stranded DNA binding protein, and incubating for a first time period to obtain a first product, wherein the first product contains a conjugate of the single-stranded DNA and the single-stranded DNA binding protein; and
purifying the first product to obtain a target product containing purified single-stranded DNA.

2. The method according to claim 1, **characterized in that** when the sample is mixed with the single-stranded DNA binding protein, the single-stranded DNA binding protein has a final concentration of 0.5 ng/µL to 500 ng/µL.

3. The method according to claim 1, **characterized in that** when the sample is mixed with the single-stranded DNA binding protein, the single-stranded DNA binding protein has a final concentration of 1 ng/µL to 200 ng/µL.

4. The method according to claim 1, **characterized in that** the first time period is 30 minutes to 12 hours.

5. The method according to claim 1, **characterized in that** the first time period is 2-4 hours.

6. The method according to claim 1, **characterized in that** the sample is mixed with the single-stranded DNA binding protein and incubated at 30-37°C.

7. The method according to claim 1, **characterized in that** the step of purifying the first product comprises:
separating and purifying the first product to obtain a second product; and
purifying the second product to at least remove the single-stranded DNA binding protein, so as to obtain the target product containing the purified single-stranded DNA.

8. The method according to claim 7, **characterized in that** the first product is separated and purified by affinity chromatography, ion exchange chromatography or gel filtration chromatography.

9. The method according to claim 8, **characterized in that** the first product is separated and purified by the affinity chromatography.

10. The method according to claim 9, **characterized in that** the single-stranded DNA binding protein carries a purification tag.

11. The method according to claim 10, **characterized in that** the purification tag is a polyhistidine tag, a glutathione-S-transferase tag, a FLAG tag or a Strep-tagII tag.

12. The method according to claim 7, **characterized in that** the second product is purified by a process selected from at least one of the following: phenol chloroform extraction, DNA adsorption spin column recovery, plasmid extraction ion exchange column recovery, isopropanol precipitation, ethanol precipitation and molecular sieve chromatography.

13. The method according to claim 1, further comprising performing PCR amplification and DNA sequencing on the target product to verify whether the target product contains a complete sequence of the single-stranded DNA.

14. The method according to claim 1, **characterized in that** the single-stranded DNA binding protein comprises a single-stranded DNA binding protein derived from *Escherichia coli,* phage phi29, *Salmonella* phage Vi II-E1, *Thermus thermophilus* HB8, *Klebsiella* phage Phi K02, *Yersinia* phage Py54 or *Synechococcus* phage S-CBS2, a core functional fragment thereof, a homolog sequence thereof, or a mutant sequence thereof.

15. The method according to claim 14, **characterized in that** the single-stranded DNA binding protein comprises a sequence that has at least 80% sequence identity with an amino acid sequence set forth in SEQ ID NO: 2, 9, 10 or 11 and has a function of binding to single-stranded DNA.

16. A kit for purifying single-stranded DNA, **characterized in that** the kit comprises: a single-stranded DNA binding protein and a purification reagent.

17. The kit according to claim 16, **characterized in that** the purification reagent is an affinity chromatography purification reagent.

18. A single-stranded DNA binding protein for use in purifying single-stranded DNA.
